# EUROPEAN PATENT APPLICATION

(11) **EP 4 523 631 A1**
(43) Date of publication of application: **19.03.2025**
(21) Application number: 23803202.3
(22) Date of filing: 09.02.2023
(51) Int. Cl.: A61B 6/03, A61B 17/88, A61B 34/10

(54) **MOUNTED BODY DESIGNING ASSISTANCE DEVICE, MOUNTED BODY DESIGNING ASSISTANCE METHOD, AND MOUNTED BODY DESIGNING ASSISTANCE PROGRAM**

(30) Priority: 10.05.2022 JP 2022077803
(71) Applicant: Tohoku University, Sendai-shi, Miyagi 980-8577 (JP)
(72) Inventor: HAGIWARA, Yoshihiro, Sendai-shi, Miyagi 980-8577 (JP); DOI, Akio, Takizawa-shi, Iwate 020-0611 (JP); SUZUKI, Kazuhiro, Morioka-shi, Iwate 020-0063 (JP)
(74) Representative: Hoffmann Eitle
(86) International application number: PCT/JP2023/004361
(87) International publication number: WO 2023/218711

(57) **Abstract**

A mounted body design assistance device 10 includes a projection position information acquisition unit 104, a smooth surface information acquisition unit 105, and a mounted body shape information generation unit 106. The projection position information acquisition unit acquires projection position information representing projection positions where sample positions in a mounted body corresponding area on a reference plane are each projected onto a biological component surface in a reference direction, based on reference direction information representing the reference direction, position information representing a position of the mounted body corresponding area on the reference plane perpendicular to the reference direction, and biological component surface shape information. The smooth surface information acquisition unit acquires smooth surface information representing a smooth surface defined by using the projection positions as control points. The mounted body shape information generation unit generates mounted body shape information so that the mounted body has the smooth surface.

## Description

### Technical Field

This invention relates to a mounted body design assistance device, a mounted body design assistance method, and a mounted body design assistance program.

### Background Art

A mounted body design method is known for designing a mounted body to be mounted to a biological component surface, which is a surface of a part constituting a living body. For example, the mounted body design method described in Patent Literature 1 designs the mounted body to have an inverted surface of the biological component surface.

### Citation List

### Patent Literature

Patent Literature 1: Japanese Translation of PCT International Application No. 2021-514726A

### Summary of Invention

### Technical Problem

Incidentally, a position at which the mounted body is mounted to the biological component surface (in other words, a mounting position) and orientation of the mounted body relative to the biological component surface when the mounted body is mounted to the biological component surface (in other words, a mounting direction) vary depending on the purpose and use of the mounted body, etc. However, the above mounted body design method has a problem of difficulty to easily design the mounted body suitable for the mounting position and mounting direction.

Furthermore, a technique is known for estimating a surface of a bone including a fractured portion when the fractured portion is to be joined. Therefore, the inventors of the present application have considered using the surface of the bone estimated by the above technique as the biological component surface in the mounted body design method.

By the way, the actual surface of the bone often differs from the estimated surface of the bone. However, in the above mounted body design method, the mounted body is designed to have the inverted surface of the biological component surface. Therefore, the mounted body designed by the above mounted body design method may not sufficiently fit the actual surface of the bone. This type of problem may also occur on the biological component surface other than the surface of bone.

One purpose of this invention is to assist in designing the mounted body that can sufficiently fit the biological component surface.

### Solution to Problem

In one aspect, a mounted body design assistance device assists in designing a mounted body to be mounted to a biological component surface, which is a surface of a part constituting a living body.

The mounted body design assistance device includes a reference direction information reception unit, a position information reception unit, a projection position information acquisition unit, a smooth surface information acquisition unit, and a mounted body shape information generation unit.

The reference direction information reception unit receives reference direction information representing a reference direction that is a direction of reference with respect to the biological component surface.

The position information reception unit receives position information representing a position of a mounted body corresponding area, which is an area corresponding to the mounted body in a reference plane that is a plane perpendicular to the reference direction.

The projection position information acquisition unit acquires projection position information representing projection positions, where sample positions in the mounted body corresponding area on the reference plane are projected onto the biological component surface in the reference direction, respectively, based on biological component surface shape information representing a three-dimensional shape of the biological component surface, the received reference direction information, and the received position information.

The smooth surface information acquisition unit acquires smooth surface information representing a smooth surface that is a smooth curved surface defined by using the projection positions as control points based on the acquired projection position information.

The mounted body shape information generation unit generates mounted body shape information representing a three-dimensional shape of the mounted body so that the mounted body has at least a part of the smooth surface based on the acquired smooth surface information.

In another aspect, a mounted body design assistance method assists in designing a mounted body to be mounted to a biological component surface, which is a surface of a part constituting a living body.

The mounted body design assistance method includes
receiving reference direction information representing a reference direction that is a direction of reference with respect to the biological component surface,
receiving position information representing a position of a mounted body corresponding area, which is an area corresponding to the mounted body in a reference plane that is a plane perpendicular to the reference direction,
acquiring projection position information representing projection positions, where sample positions in the mounted body corresponding area on the reference plane are projected onto the biological component surface in the reference direction, respectively, based on biological component surface shape information representing a three-dimensional shape of the biological component surface, the received reference direction information, and the received position information,
acquiring smooth surface information representing a smooth surface that is a smooth curved surface defined by using the projection positions as control points based on the acquired projection position information, and
generating mounted body shape information representing a three-dimensional shape of the mounted body so that the mounted body has at least a part of the smooth surface based on the acquired smooth surface information.

In another aspect, a mounted body design assistance program causes a computer to execute a process for assisting in designing a mounted body to be mounted to a biological component surface, which is a surface of a part constituting a living body.

The process includes
receiving reference direction information representing a reference direction that is a direction being a reference with respect to the biological component surface,
receiving position information representing a position of a mounted body corresponding area, which is an area corresponding to the mounted body in a reference plane that is a plane perpendicular to the reference direction,
acquiring projection position information representing projection positions, where sample positions in the mounted body corresponding area on the reference plane are projected onto the biological component surface in the reference direction, respectively, based on biological component surface shape information representing a three-dimensional shape of the biological component surface, the received reference direction information, and the received position information,
acquiring smooth surface information representing a smooth surface that is a smooth curved surface defined by using the projection positions as control points based on the acquired projection position information, and
generating mounted body shape information representing a three-dimensional shape of the mounted body so that the mounted body has at least a part of the smooth surface based on the acquired smooth surface information.

### Advantageous Effects of Invention

The design of the mounted body that can sufficiently fit the biological component surface can be assisted.

### Brief Description of Drawings

Fig. 1 is a block diagram illustrating a configuration of a mounted body design assistance device of a first embodiment.
Fig. 2 is a block diagram illustrating a function of the mounted body design assistance device of the first embodiment.
Fig. 3 is an explanatory diagram illustrating a three-dimensional shape of a biological component surface displayed by the mounted body design assistance device of the first embodiment.
Fig. 4 is an explanatory diagram illustrating the three-dimensional shape of the biological component surface displayed by the mounted body design assistance device of the first embodiment.
Fig. 5 is an explanatory diagram illustrating a relationship between sample positions and projection positions used by the mounted body design assistance device of the first embodiment.
Fig. 6 is an explanatory diagram illustrating the sample positions and the projection positions used by the mounted body design assistance device of the first embodiment.
Fig. 7 is an explanatory diagram illustrating the projection positions and auxiliary positions used by the mounted body design assistance device of the first embodiment.
Fig. 8 is an explanatory diagram illustrating the projection positions and the auxiliary positions used by the mounted body design assistance device of the first embodiment.
Fig. 9 is an explanatory diagram illustrating the projection positions, the auxiliary positions, and a smooth surface used by the mounted body design assistance device of the first embodiment.
Fig. 10 is an explanatory diagram illustrating the smooth surface and a first moving solid body used by the mounted body design assistance device of the first embodiment.
Fig. 11 is an explanatory diagram illustrating the smooth surface, the first moving solid body, and a second moving solid body used by the mounted body design assistance device of the first embodiment.
Fig. 12 is an explanatory diagram illustrating an intersection solid body used by the mounted body design assistance device of the first embodiment.
Fig. 13 is an explanatory diagram illustrating the intersection solid body used by the mounted body design assistance device of the first embodiment.
Fig. 14 is a flowchart illustrating a process executed by the mounted body design assistance device of the first embodiment.
Fig. 15 is a flowchart illustrating a process executed by the mounted body design assistance device of the first embodiment.
Fig. 16 is a flowchart illustrating a process executed by the mounted body design assistance device of the first embodiment.
Fig. 17 is a flowchart illustrating a process executed by the mounted body design assistance device of the first embodiment.

### Description of Embodiments

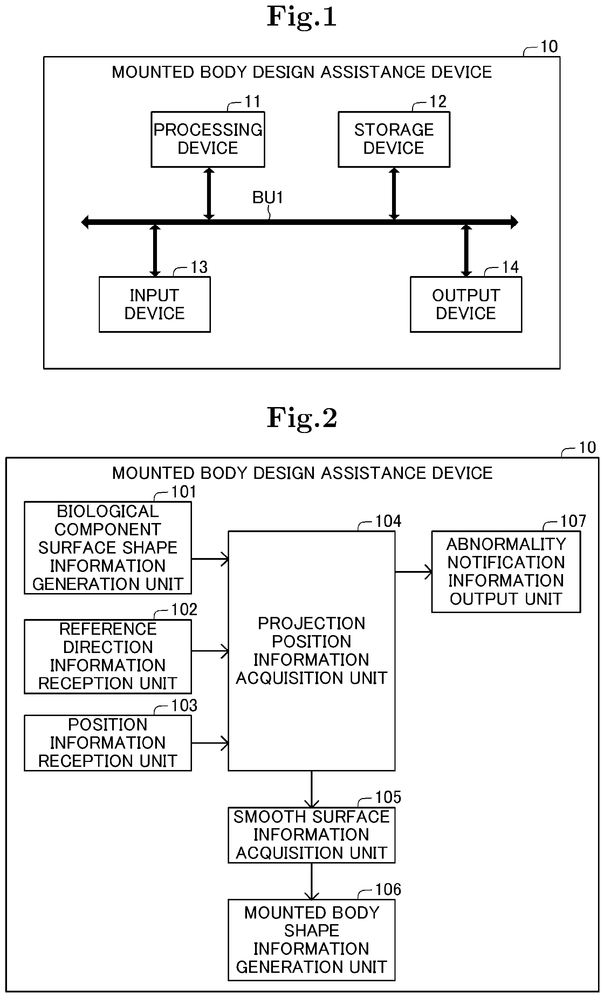

Hereinafter, each embodiment of a mounted body design assistance device, a mounted body design assistance method, and a mounted body design assistance program of this invention will be described with reference to Figs. 1 to 17.

### <First Embodiment>

### (Overview)

A mounted body design assistance device of a first embodiment assists in designing a mounted body to be mounted to a biological component surface, which is a surface of a part constituting a living body.

The mounted body design assistance device includes a reference direction information reception unit, a position information reception unit, a projection position information acquisition unit, a smooth surface information acquisition unit, and a mounted body shape information generation unit.

The reference direction information reception unit receives reference direction information representing a reference direction that is a direction of reference with respect to the biological component surface.

The position information reception unit receives position information representing a position of a mounted body corresponding area, which is an area corresponding to the mounted body in a reference plane that is a plane perpendicular to the reference direction.

The projection position information acquisition unit acquires projection position information representing projection positions, where sample positions in the mounted body corresponding area on the reference plane are projected onto the biological component surface in the reference direction, respectively, based on biological component surface shape information representing a three-dimensional shape of the biological component surface, the received reference direction information, and the received position information.

The smooth surface information acquisition unit acquires smooth surface information representing a smooth surface that is a smooth curved surface defined by using the projection positions as control points based on the acquired projection position information.

The mounted body shape information generation unit generates mounted body shape information representing a three-dimensional shape of the mounted body so that the mounted body has at least a part of the smooth surface based on the acquired smooth surface information.

According to this, a user of the mounted body design assistance device can easily generate the mounted body shape information by inputting the reference direction information and the position information to the mounted body design assistance device. Therefore, the mounted body can be easily designed according to the reference direction and the position of the mounted body corresponding area.

Moreover, according to the mounted body design assistance device, the mounted body shape information is generated so that the mounted body has at least a part of the smooth surface. Therefore, the design of the mounted body that can sufficiently fit the biological component surface can be assisted.

Next, the mounted body design assistance device of the first embodiment will be described in more detail.

### (Configuration)

As illustrated in Fig. 1, the mounted body design assistance device 10 includes a processing device 11, a storage device 12, an input device 13, and an output device 14, which are connected to each other via a bus BU1. For example, the mounted body design assistance device 10 is constituted by a computer (in other words, information processing device). The computer may be a server-type computer, a desktop-type computer, a laptop-type computer, or a tablet-type computer. Alternatively, the computer may be at least a part of a stationary game console, a portable game console, a television set, a smartphone, or the like. Alternatively, the mounted body design assistance device 10 may be composed of multiple devices communicatively connected to each other.

The processing device 11 controls the storage device 12, the input device 13, and the output device 14 by executing a program stored in the storage device 12. In this way, the processing device 11 realizes functions described below.

In this example, the processing device 11 is a Central Processing Unit (CPU). The processing device 11 may include a Micro Processing Unit (MPU), a Graphics Processing Unit (GPU), or a Digital Signal Processor (DSP) instead of or in addition to a CPU.

In this example, the storage device 12 includes a volatile memory and a non-volatile memory. For example, the storage device 12 includes at least one of a Random Access Memory (RAM), a Read Only Memory (ROM), a semiconductor memory, an organic memory, a Hard Disk Drive (HDD), and a Solid State Drive (SSD).

The input device 13 inputs information from the outside of the mounted body design assistance device 10. In this example, the input device 13 includes a keyboard and a mouse. The input device 13 may include a microphone.

The output device 14 outputs information to the outside of the mounted body design assistance device 10. In this example, the output device 14 includes a display. The output device 14 may include a speaker. Alternatively, the mounted body design assistance device 10 may include a touch panel display that constitutes both the input device 13 and the output device 14.

### (Function)

The mounted body design assistance device 10 assists in designing a mounted body to be mounted to a biological component surface, which is a surface of a part constituting a living body. In this example, the part constituting the living body is a bone including a fractured portion. In this example, the mounted body is a bone plate. The part constituting the living body may be a bone not including any fractured portions, or skin. Alternatively, for example, the mounted body may be an appliance, or an exercise aid for assisting exercise.

As illustrated in Fig. 2, the functions of the mounted body design assistance device 10 include a biological component surface shape information generation unit 101, a reference direction information reception unit 102, a position information reception unit 103, a projection position information acquisition unit 104, a smooth surface information acquisition unit 105, a mounted body shape information generation unit 106, and an abnormality notification information output unit 107.

The biological component surface shape information generation unit 101 generates biological component surface shape information representing a three-dimensional shape of the biological component surface. In this example, the biological component surface may be represented as a bone isosurface.

In this example, the biological component surface shape information generation unit 101 generates the biological component surface shape information based on biological component shape information representing a three-dimensional shape of the part constituting the living body. For example, the biological component shape information may be acquired using a technique called Computed Tomography (CT), Magnetic Resonance Imaging (MRI), Computed Radiography (CR), or Digital Radiography (DR). For example, the biological component shape information may be information that complies with a standard called Digital Imaging and Communications in Medicine (DICOM).

In this example, the biological component shape information is stored in advance in the storage device 12. The biological component shape information may be input to the mounted body design assistance device 10 via a recording medium or a communication line.

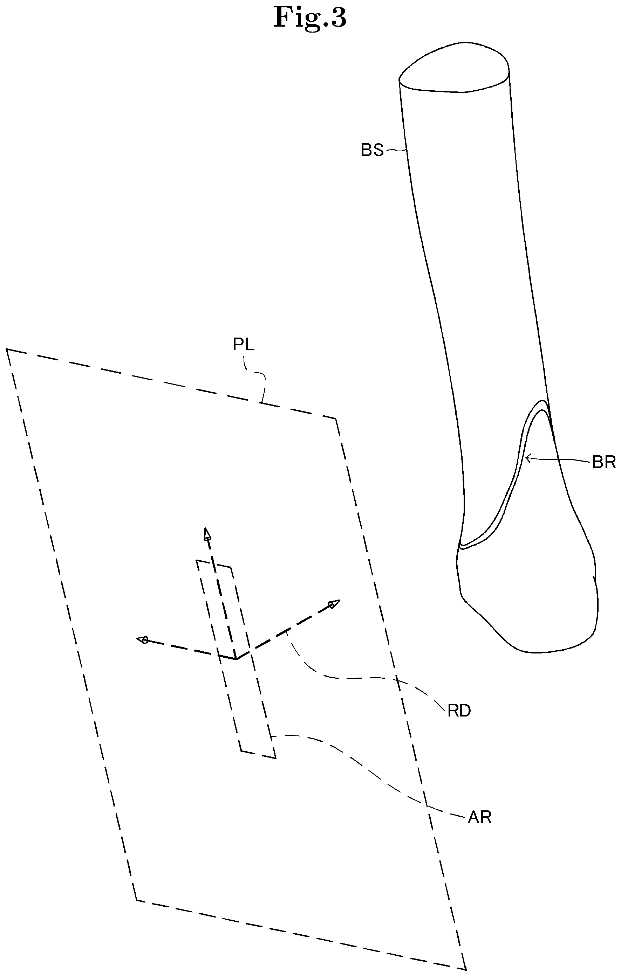

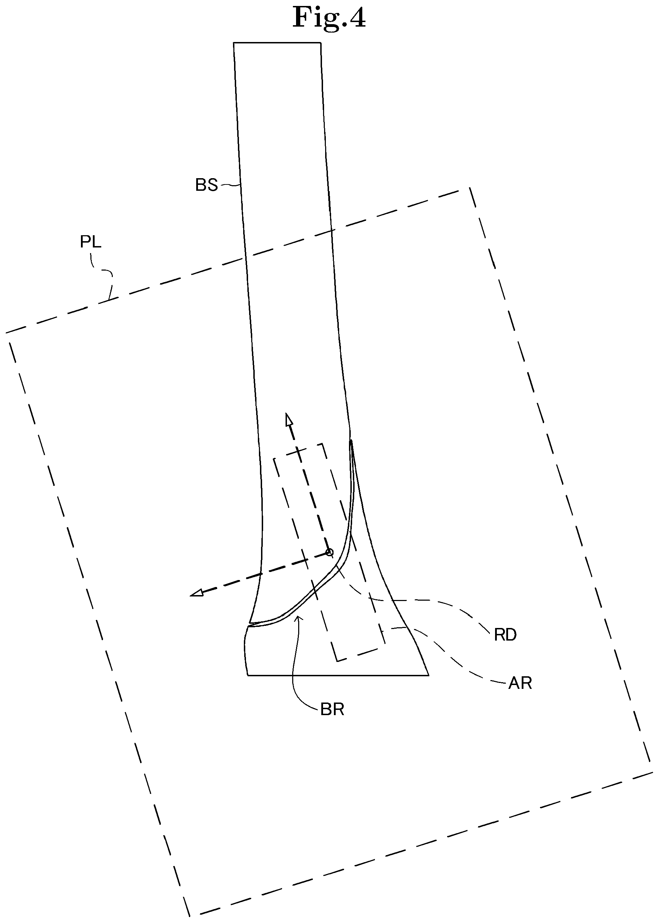

The reference direction information reception unit 102 and the position information reception unit 103 display the three-dimensional shape of the biological component surface BS represented by the biological component surface shape information generated by the biological component surface shape information generation unit 101 via the output device 14, as illustrated in Figs. 3 and 4. Figs. 3 and 4 illustrate the biological component surface BS viewed from different directions from each other. The biological component surface BS includes the fractured portion BR.

The reference direction information reception unit 102 receives reference direction information representing a reference direction RD that is a direction of reference with respect to the biological component surface BS.

The position information reception unit 103 receives position information representing a position of a mounted body corresponding area AR, which is an area corresponding to the mounted body in a reference plane PL that is a plane perpendicular to the reference direction RD.

The mounted body corresponding area AR is rectangular. In this example, the mounted body corresponding area AR is the smallest rectangular shape that includes the shape of the mounted body. The shape of the mounted body may be set based on information input by the user of the mounted body design assistance device 10 via the input device 13. Alternatively, the shape of the mounted body may be set in advance.

In this example, the position information and the reference direction information are input by the user of the mounted body design assistance device 10 via the input device 13.

The projection position information acquisition unit 104 acquires projection position information based on the biological component surface shape information generated by the biological component surface shape information generation unit 101, the reference direction information received by the reference direction information reception unit 102, and the position information received by the position information reception unit 103.

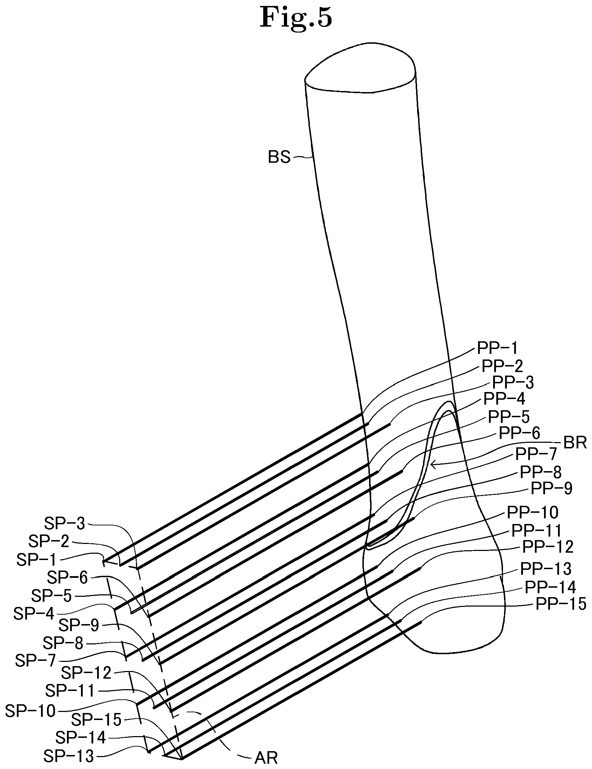

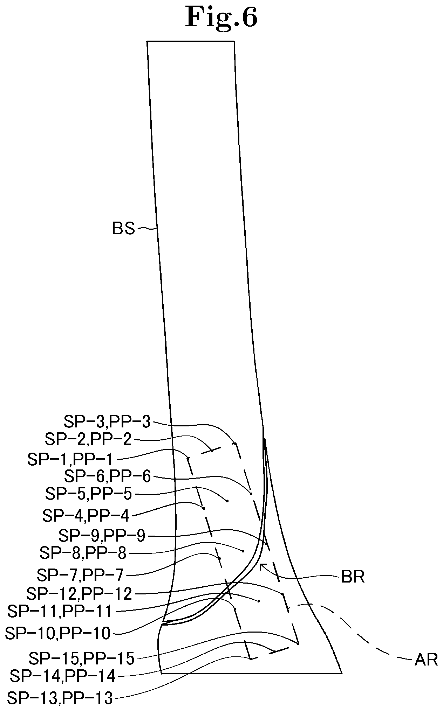

As illustrated in Figs. 5 and 6, the projection position information represents N projection positions PP-1 to PP-N, where N sample positions SP-1 to SP-N in the mounted body corresponding area AR on the reference plane PL are projected onto the biological component surface BS in the reference direction RD, respectively. N represents an integer of 4 or more. In this example, N represents 15.

In this example, the N sample positions SP-1 to SP-N are located in a grid pattern. In this example, the N sample positions SP-1 to SP-N are equally spaced at a predetermined short side spacing in the short side direction of the mounted body corresponding area AR, and are equally spaced at a predetermined long side spacing in the long side direction of the mounted body corresponding area AR. In this example, the short side spacing and the long side spacing are different from each other. Alternatively, the short side spacing and the long side spacing may be equal to each other.

For example, the short side spacing may be set to be longer as the length of the short side of the mounted body corresponding area AR becomes longer. For example, the long side spacing may be set to be longer as the length of the long side of the mounted body corresponding area AR becomes longer. Alternatively, for example, the long side spacing may be set to be longer as the length of the short side of the mounted body corresponding area AR becomes longer.

Alternatively, the short side spacing and the long side spacing may be set based on information input by the user of the mounted body design assistance device 10 via the input device 13.

In this example, the projection position information acquisition unit 104 executes a first projection position correction process, a second projection position correction process, and a third projection position correction process. Alternatively, the projection position information acquisition unit 104 may not execute at least a part of the first projection position correction process, the second projection position correction process, and the third projection position correction process.

First, the first projection position correction process will be described.

In this example, in the first projection position correction process, for each of the N sample positions SP-1 to SP-N, when a magnitude of a difference between a reference direction component, which is a component in the reference direction RD of the projection position PP-n for a focused sample position SP-n being the sample position SP-n, and a reference value is greater than a threshold, the projection position information acquisition unit 104 corrects the projection position PP-n for the focused sample position SP-n based on adjacent projection positions, each of which is a projection position for a sample position adjacent to the focused sample position SP-n. n represents an integer of 1 to N.

In this example, the projection position PP-n is represented using a Cartesian coordinate system, which has mutually perpendicular x-axis, y-axis, and z-axis with the origin coinciding with the center of gravity of the mounted body corresponding area AR and where the positive direction of the z-axis coincides with the reference direction RD. Thus, in this example, the reference direction component corresponds to the coordinate on the z-axis of the projection position PP-n. In other words, a magnitude of the reference direction component corresponds to a distance of the projection position PP-n from the reference plane PL. Alternatively, the origin of the Cartesian coordinate system may have a position different from the center of gravity of the mounted body corresponding area AR.

Specifically, for each of the N sample positions SP-1 to SP-N, the projection position information acquisition unit 104 acquires the reference direction component of the projection position PP-n for the focused sample position SP-n, which is the sample position SP-n.

Then, the projection position information acquisition unit 104 determines the reference value, a first threshold, and a second threshold based on the reference direction components of the N projection positions PP-1 to PP-N acquired for the N sample positions SP-1 to SP-N, respectively.

In this example, the reference value is the average value of the reference direction components of the N projection positions PP-1 to PP-N. The first threshold is the standard deviation of the reference direction components of the N projection positions PP-1 to PP-N multiplied by a predetermined first coefficient. In this example, the first coefficient is a positive real number (for example, 3.0). The second threshold is the standard deviation of the reference direction components for the N projection positions PP-1 to PP-N multiplied by a predetermined second coefficient. In this example, the second coefficient is a positive real number (for example, 1.5) smaller than the first coefficient. The second coefficient may be equal to the first coefficient.

Next, the projection position information acquisition unit 104 acquires, for each of the N sample positions SP-1 to SP-N, a reference direction component difference, which is a value acquired by subtracting the determined reference value from the reference direction component of the projection position PP-n for the focused sample position SP-n being the sample position SP-n.

Next, the projection position information acquisition unit 104 determines, for each of the N sample positions SP-1 to SP-N, whether or not the projection position PP-n for the focused sample position SP-n, which is the sample position SP-n, needs to be corrected based on the reference direction component difference acquired for the focused sample position SP-n.

In this example, in the case of the reference direction component difference acquired for the focused sample position SP-n having a negative value, when the magnitude of the reference direction component difference is greater than the determined first threshold, the projection position information acquisition unit 104 determines that the projection position PP-n for the focused sample position SP-n needs to be corrected, whereas when the magnitude of the reference direction component difference is equal to or less than the determined first threshold, the projection position information acquisition unit 104 determines that the projection position PP-n for the focused sample position SP-n does not need to be corrected.

In this example, the reference direction component difference acquired for the focused sample position SP-n having a negative value corresponds to the projection position PP-n for the focused sample position SP-n being closer to the reference plane PL than a parallel plane. In this example, the parallel plane is a plane that is parallel to the reference plane PL and passes through a position whose coordinate on the z-axis has the reference value.

Furthermore, in this example, in the case of the reference direction component difference acquired for the focused sample position SP-n having a positive value, when the magnitude of the reference direction component difference is greater than the determined second threshold, the projection position information acquisition unit 104 determines that the projection position PP-n for the focused sample position SP-n needs to be corrected, whereas when the magnitude of the reference direction component difference is equal to or less than the determined second threshold, the projection position information acquisition unit 104 determines that the projection position PP-n for the focused sample position SP-n does not need to be corrected.

In this example, the reference direction component difference acquired for the focused sample position SP-n having a positive value corresponds to the projection position PP-n for the focused sample position SP-n being farther from the reference plane PL than the parallel plane.

In this example, for each sample position (in other words, correction necessity sample position) SP-n for a projection position (in other words, correction necessity projection position) PP-n that is determined to need to be corrected, the projection position information acquisition unit 104 corrects the projection position PP-n for the focused sample position SP-n, which is the sample position SP-n, by setting the average value of the reference direction components of projection positions (in other words, correction unnecessity projection positions) that is determined not to need to be corrected among extended adjacent projection positions for the focused sample position SP-n as the reference direction component of the projection position PP-n for the focused sample position SP-n.

In this example, the extended adjacent projection positions for the focused sample position SP-n consist of direct adjacent projection positions, which are projection positions for direct adjacent sample positions being sample positions adjacent to the focused sample position SP-n, and indirect adjacent projection positions, which are projection positions for indirect adjacent sample positions being sample positions adjacent to each of two sample positions adjacent to the focused sample position SP-n.

In this example, when the focused sample position SP-n has a position other than four sides that constitute the outer edge of the mounted body corresponding area AR, the number of the direct adjacent projection positions is four, and the number of the indirect adjacent projection positions is four. For example, the direct adjacent projection positions for the focused sample position SP-5 consist of the projection position PP-2, the projection position PP-4, the projection position PP-6, and the projection position PP-8. The indirect adjacent projection positions for the focused sample position SP-5 consist of the projection position PP-1, the projection position PP-3, the projection position PP-7, and the projection position PP-9.

In this example, when the focused sample position SP-n has a position other than corners that constitute the outer edge of the mounted body corresponding area AR among sides that constitute the outer edge of the mounted body corresponding area AR, the number of the direct adjacent projection positions is three, and the number of the indirect adjacent projection positions is two. For example, the direct adjacent projection positions for the focused sample position SP-7 consist of the projection position PP-4, the projection position PP-8, and the projection position PP-10. The indirect adjacent projection positions for the focused sample position SP-7 consist of the projection position PP-5 and the projection position PP-11.

In this example, when the focused sample position SP-n has a position at a corner that constitutes the outer edge of the mounted body corresponding area AR, the number of the direct adjacent projection positions is two, and the number of the indirect adjacent projection positions is one. For example, the direct adjacent projection positions for the focused sample position SP-3 consist of the projection position PP-2 and the projection position PP-6. The indirect adjacent projection position for the focused sample position SP-3 consists of the projection position PP-5.

In this way, the projection position information acquisition unit 104 executes the first projection position correction process.

Next, the second projection position correction process will be described.

In this example, in the second projection position correction process, the projection position information acquisition unit 104 assigns one of two values being a first value or a second value to each of the N sample positions SP-1 to SP-N by binarizing the reference direction components of the N projection positions PP-1 to PP-N for the N sample positions SP-1 to SP-N. Furthermore, in the second projection position correction process, for each of the N sample positions SP-1 to SP-N, when a value different from a value assigned to the focused sample position SP-n, which is the sample position SP-n, is assigned to all sample positions adjacent to the focused sample position SP-n, the projection position information acquisition unit 104 corrects the projection position PP-n for the focused sample position SP-n based on adjacent projection positions, each of which is a projection position for a sample position adjacent to the focused sample position SP-n.

Specifically, the projection position information acquisition unit 104 acquires, for each of the N sample positions SP-1 to SP-N, the reference direction component of the projection position PP-n for the focused sample position SP-n, which is the sample position SP-n.

Next, the projection position information acquisition unit 104 determines a binarization threshold for binarizing the reference direction components of the N projection positions PP-n based on the reference direction components of the N projection positions PP-1 to PP-N acquired for the N sample positions SP-1 to SP-N, respectively. In this example, the binarization threshold is determined using a method called Otsu's binarization method (in other words, Otsu's method).

Next, for each of the N sample positions SP-1 to SP-N, the projection position information acquisition unit 104 assigns the first value to the focused sample position SP-n, which is the sample position SP-n, when the reference direction component of the projection position PP-n for the focused sample position SP-n is smaller than the binarization threshold, whereas the projection position information acquisition unit 104 assigns the second value to the focused sample position SP-n when the reference direction component of the projection position PP-n for the focused sample position SP-n is equal to or greater than the binarization threshold. In this example, the second value is greater than the first value. For example, the first value may be 0, and the second value may be 1.

Next, the projection position information acquisition unit 104 corrects, for each of the N sample positions SP-1 to SP-N, the projection position PP-n for the focused sample position SP-n, which is the sample position SP-n, by setting the average value of the reference direction components of all extended adjacent projection positions for the focused sample position SP-n as the reference direction component of the projection position PP-n for the focused sample position SP-n when a value assigned to the focused sample position SP-n is isolated.

In this example, the isolation of a value assigned to the focused sample position SP-n corresponds to a value different from a value assigned to the focused sample position SP-n being assigned to all of the direct adjacent sample positions for the focused sample position SP-n. Alternatively, the isolation of a value assigned to the focused sample position SP-n may correspond to a value different from a value assigned to the focused sample position SP-n being assigned to all of the direct adjacent sample positions and indirect adjacent sample positions for the focused sample position SP-n.

In this way, the projection position information acquisition unit 104 executes the second projection position correction process.

Next, the third projection position correction process will be described.

In this example, in the third projection position correction process, the projection position information acquisition unit 104 determines, for each of the N sample positions SP-1 to SP-N, the projection position PP-n for the focused sample position SP-n, which is the sample position SP-n, based on projection positions for sample positions adjacent to the focused sample position SP-n when the biological component surface BS is not present on a straight line that passes through the focused sample position SP-n and extends in the reference direction RD.

Specifically, for each of the N sample positions SP-1 to SP-N, the projection position information acquisition unit 104 determines whether or not the biological component surface BS is present on the straight line (in other words, projection line) that passes through the focused sample position SP-n, which is the sample position SP-n, and extends in the reference direction RD.

Next, for each sample position (in other words, absence sample position) SP-n, for which it is determined that the biological component surface BS is not present on the projection line, the projection position information acquisition unit 104 determines the projection position PP-n for the focused sample position SP-n, which is the sample position SP-n, by setting the average value of the reference direction components of projection positions (in other words, present projection positions), for which it is determined that the biological component surface BS is present on the projection line, among the extended adjacent projection positions for the focused sample position SP-n as the reference direction component of the projection position PP-n for the focused sample position SP-n.

In this way, the projection position information acquisition unit 104 executes the third projection position correction process.

The projection position information acquisition unit 104 acquires projection position information representing the N projection positions PP-1 to PP-N corrected by executing the first projection position correction process, the second projection position correction process, and the third projection position correction process.

The smooth surface information acquisition unit 105 acquires auxiliary position information based on the projection position information acquired by the projection position information acquisition unit 104. The auxiliary position information represents M auxiliary positions. M represents an integer of 2 or more. In this example, M represents the number (in this example, 20), which is the number (in this example, 16) of sample positions SP-n located on the four sides that constitute the outer edge of the mounted body corresponding area AR plus the number (in this example, 4) of sample positions SP-n located at the corners that constitute the outer edge of the mounted body corresponding area AR.

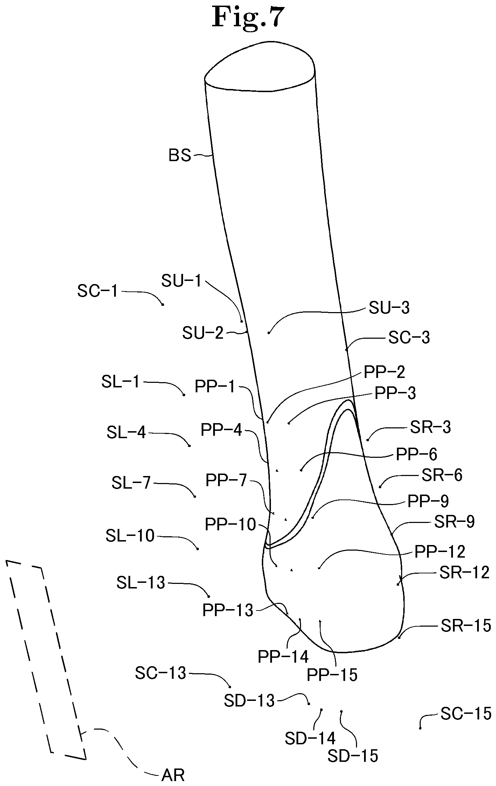

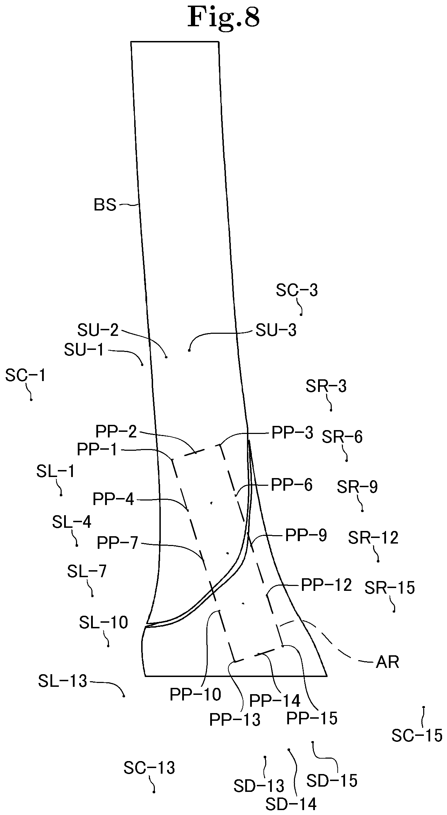

In this example, as illustrated in Figs. 7 and 8, the M auxiliary positions include six short side extended auxiliary positions SU-1, SU-2, SU-3, SD-13, SD-14, SD-15, ten long side extended auxiliary positions SR-3, SR-6, SR-9, SR-12, SR-15, SL-1, SL-4, SL-7, SL-10, SL-13, and four corner extended auxiliary positions SC-1, SC-3, SC-13, SC-15.

In this example, the short side extended auxiliary position has a position acquired by moving a projection position PP-n for a sample position SP-n located on the two short sides that constitute the outer edge of the mounted body corresponding area AR in a short side extended direction by a short side extended distance. The short side extended direction is a direction perpendicular to each of the short side, on which the sample position SP-n for the projection position PP-n to be moved is located, and the reference direction RD, and is directed outward for the mounted body corresponding area AR.

In this example, the short side extended distance is longer than the interval between sample positions SP-n in the short side extended direction (in other words, long side spacing). In this example, the short side extended distance is set to be longer as the length of the short side of the mounted body corresponding area AR becomes longer, and to be longer as the length of the long side of the mounted body corresponding area AR becomes longer.

For example, the three short side extended auxiliary positions SU-1, SU-2, SU-3 have positions acquired by moving the three projection positions PP-1, PP-2, PP-3 for the three sample positions SP-1, SP-2, SP-3 located on the short side that constitutes the outer edge of the mounted body corresponding area AR in the short side extended direction by the short side extended distance, respectively. Therefore, the reference direction components of the three short side extended auxiliary positions SU-1, SU-2, SU-3 respectively coincide with the reference direction components of the three projection positions PP-1, PP-2, PP-3.

In this example, the long side extended auxiliary position has a position acquired by moving a projection position PP-n for a sample position SP-n located on the two long sides that constitute the outer edge of the mounted body corresponding area AR in a long side extended direction by a long side extended distance. The long side extended direction is a direction perpendicular to each of the long side, on which the sample position SP-n for the projection position PP-n to be moved is located, and the reference direction RD, and is directed outward for the mounted body corresponding area AR.

In this example, the long side extended distance is longer than the interval between sample positions SP-n in the long side extended direction (in other words, short side spacing). In this example, the long side extended distance is set to be longer as the length of the short side of the mounted body corresponding area AR becomes longer, and to be longer as the length of the long side of the mounted body corresponding area AR becomes longer. In this example, the long side extended distance is longer than the short side extended distance.

For example, the five long side extended auxiliary positions SR-3, SR-6, SR-9, SR-12, SR-15 have positions acquired by moving the five projection positions PP-3, PP-6, PP-9, PP-12, PP-15 for the five sample positions SP-3, SP-6, SP-9, SP-12, SP-15 located on the long side that constitutes the outer edge of the mounted body corresponding area AR in the long side extended direction by the long side extended distance, respectively. Therefore, the reference direction components of the five long side extended auxiliary positions SR-3, SR-6, SR-9, SR-12, SR-15 respectively coincide with the reference direction components of the five projection positions PP-3, PP-6, PP-9, PP-12, PP-15.

In this example, the corner extended auxiliary position has a position acquired by moving a projection position PP-n for a sample position SP-n located on the four corners that constitute the outer edge of the mounted body corresponding area AR in the short side extended direction by the short side extended distance and in the long side extended direction by the long side extended distance.

For example, the corner extended auxiliary position SC-13 has a position acquired by moving the projection position PP-13 for the sample position SP-13, which is located at the corner that constitutes the outer edge of the mounted body corresponding area AR, by the short side extended distance in the short side extended direction and by the long side extended distance in the long side extended direction. Therefore, the reference direction component of the corner extended auxiliary position SC-13 coincides with the reference direction component of the projection position PP-13.

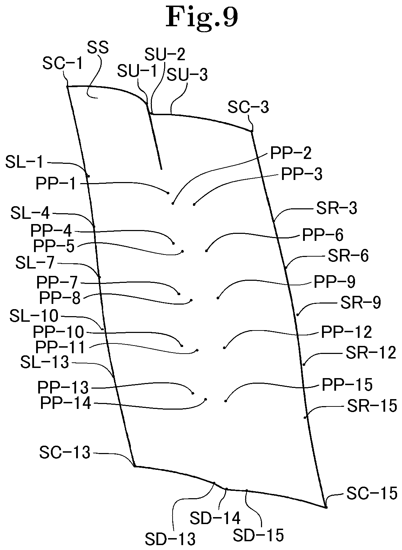

As illustrated in Fig. 9, the smooth surface information acquisition unit 105 acquires smooth surface information representing a smooth surface SS that is a smooth curved surface defined by using the N projection positions PP-1 to PP-N represented by the projection position information acquired by the projection position information acquisition unit 104 and the M auxiliary positions SU-1, SU-2, SU-3, SD-13, SD-14, SD-15, SR-3, SR-6, SR-9, SR-12, SR-15, SL-1, SL-4, SL-7, SL-10, SL-13, SC-1, SC-3, SC-13, SC-15 represented by acquired correction position information as control points.

In this example, the smooth surface SS is a B-Spline surface. The smooth surface may be a spline surface other than a B-spline surface (for example, a NURBS (Non-Uniform Rational B-Spline) surface), or a Bezier surface.

Alternatively, the smooth surface information acquisition unit 105 may acquire the smooth surface information representing a smooth surface SS that is a smooth curved surface defined by using only the N projection positions PP-1 to PP-N as control points, without using the auxiliary positions as control points.

The mounted body shape information generation unit 106 generates mounted body shape information representing a three-dimensional shape of the mounted body so that the mounted body has at least a part (for example, a part or all) of the smooth surface SS represented by the smooth surface information based on the smooth surface information acquired by the smooth surface information acquisition unit 105.

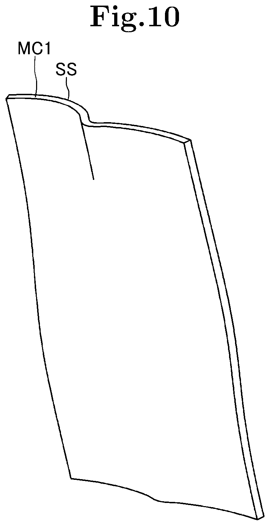

Specifically, as illustrated in Fig. 10, the mounted body shape information generation unit 106 generates first moving solid body information representing a three-dimensional shape of a first moving solid body MC1 formed by moving the smooth surface SS by a first distance along the reference direction RD (in this example, in the opposite direction to the reference direction RD).

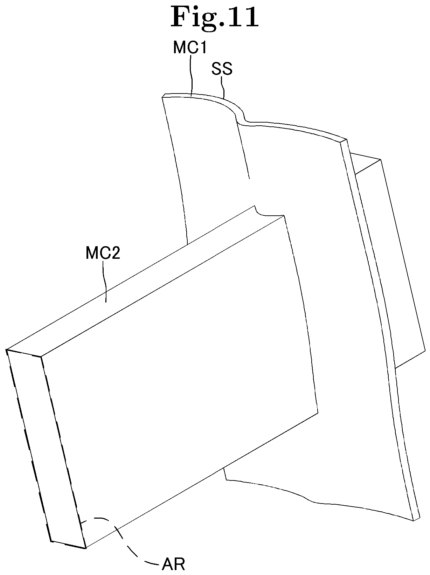

Next, as illustrated in Fig. 11, the mounted body shape information generation unit 106 generates second moving solid body information representing a three-dimensional shape of a second moving solid body MC2 formed by moving the mounted body corresponding area AR along the reference direction RD (in this example, toward the reference direction RD) by a second distance longer than the first distance.

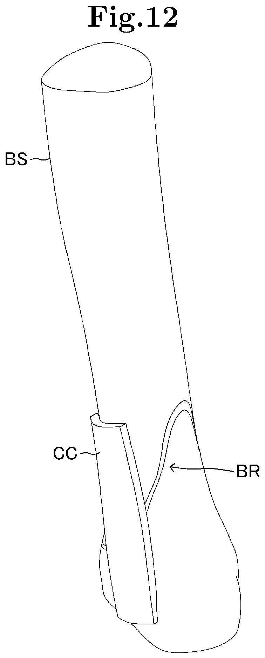

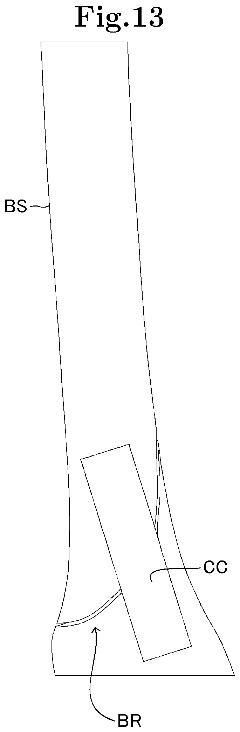

Next, as illustrated in Figs. 12 and 13, the mounted body shape information generation unit 106 generates intersection solid body information representing a three-dimensional shape of an intersection solid body CC generated by intersecting the first moving solid body MC1 and the second moving solid body MC2 based on the generated first moving solid body information and second moving solid body information.

Then, the mounted body shape information generation unit 106 generates mounted body shape information based on the generated intersection solid body information. In this example, the mounted body shape information generation unit 106 generates the mounted body shape information, which represents a three-dimensional shape in which at least a part of corners of the intersection solid body CC represented by the intersection solid body information have been rounded (in other words, at least a part of corners of the intersection solid body CC have been filleted) as a three-dimensional shape of the mounted body. Alternatively, the mounted body shape information generation unit 106 may generate the mounted body shape information, which represents a three-dimensional shape of the intersection solid body CC represented by the intersection solid body information as a three-dimensional shape of the mounted body.

The abnormality notification information output unit 107 determines whether or not a process abnormality condition is satisfied based on the projection position information acquired by the projection position information acquisition unit 104. In this example, the abnormality notification information output unit 107 makes the above determination based on the projection position information acquired before executing the first projection position correction process, the second projection position correction process, and the third projection position correction process.

In this example, the process abnormality condition is that an absence ratio is greater than a predetermined abnormality threshold. The absence ratio is a ratio of the number of sample positions SP-n, each of which a straight line (in other words, a projection line) extending in the reference direction RD passes through, the biological component surface BS not being present on the straight line, among the N sample positions SP-1 to SP-N to the total number (in this example, N) of the N sample positions SP-1 to SP-N. In this example, the abnormality threshold is a real number greater than 0 and less than 1.

When the process abnormality condition is satisfied, the abnormality notification information output unit 107 outputs, via the output device 14, abnormality notification information indicating that the mounted body shape information cannot be generated normally.

Alternatively, the functions of the mounted body design assistance device 10 may not include the abnormality notification information output unit 107.

The description of the functions of the mounted body design assistance device 10 may be supplemented by the following description of an operation of the mounted body design assistance device 10.

### (Operation)

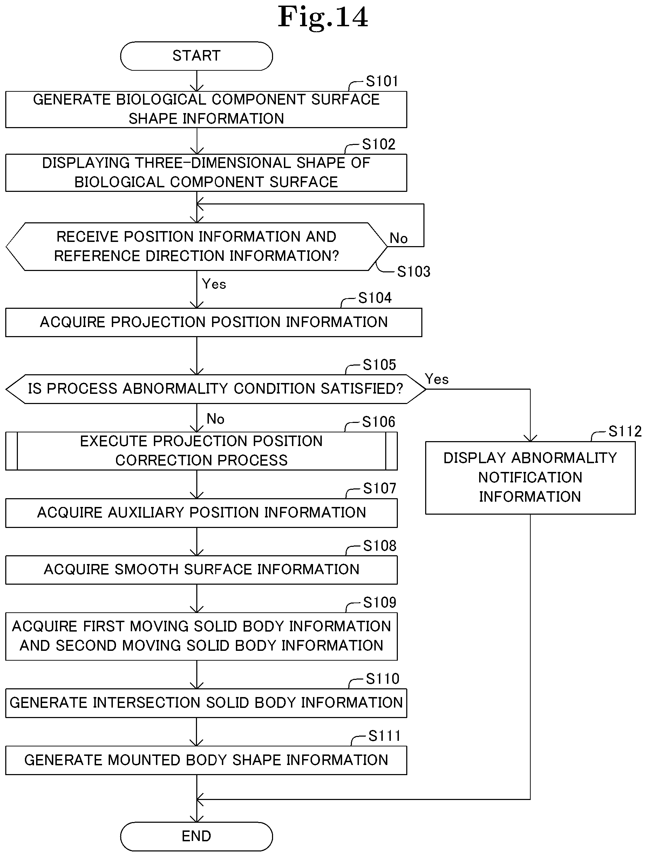

Next, the operation of the mounted body design assistance device 10 will be described with reference to Figs. 14 to 17. The mounted body design assistance device 10 executes a process illustrated in Fig. 14 in accordance with an execution instruction input by the user of the mounted body design assistance device 10 via the input device 13.

First, the mounted body design assistance device 10 generates the biological component surface shape information (step S101 in Fig. 14).

Next, the mounted body design assistance device 10 displays, via the output device 14, the three-dimensional shape of the biological component surface BS represented by the biological component surface shape information generated in step S101 (step S102 in Fig. 14).

Next, the mounted body design assistance device 10 waits until receiving the position information that represents the position of the mounted body corresponding area AR on the reference plane PL, and the reference direction information that represents the reference direction RD ("No" route of step S103 in Fig. 14).

When the position information and the reference direction information are input to the mounted body design assistance device 10 via the input device 13, the mounted body design assistance device 10 receives the position information and the reference direction information, determines "Yes" in step S103, and proceeds to step S104.

Next, the mounted body design assistance device 10 acquires the projection position information based on the biological component surface shape information generated in step S101, and the position information and reference direction information received in step S103 (step S104 in Fig. 14).

Next, the mounted body design assistance device 10 determines whether or not the process abnormality condition is satisfied based on the projection position information acquired in step S104 (step S105 in Fig. 14).

When the process abnormality condition is not satisfied, the mounted body design assistance device 10 determines "No" in step S105, and proceeds to step S106.

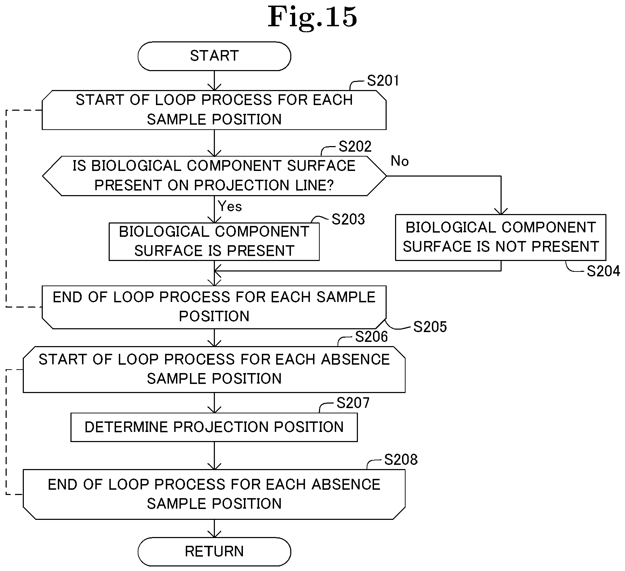

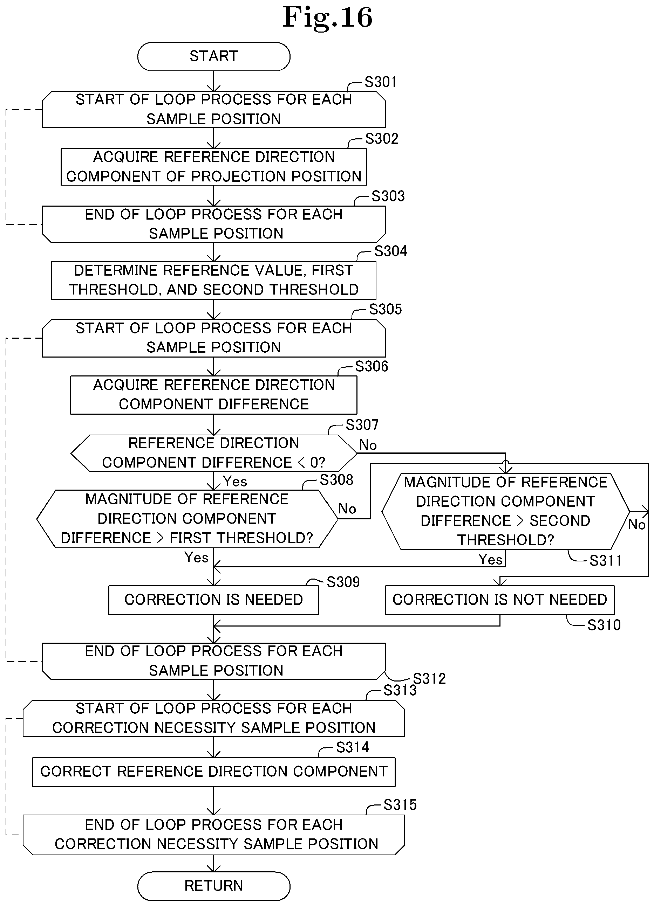

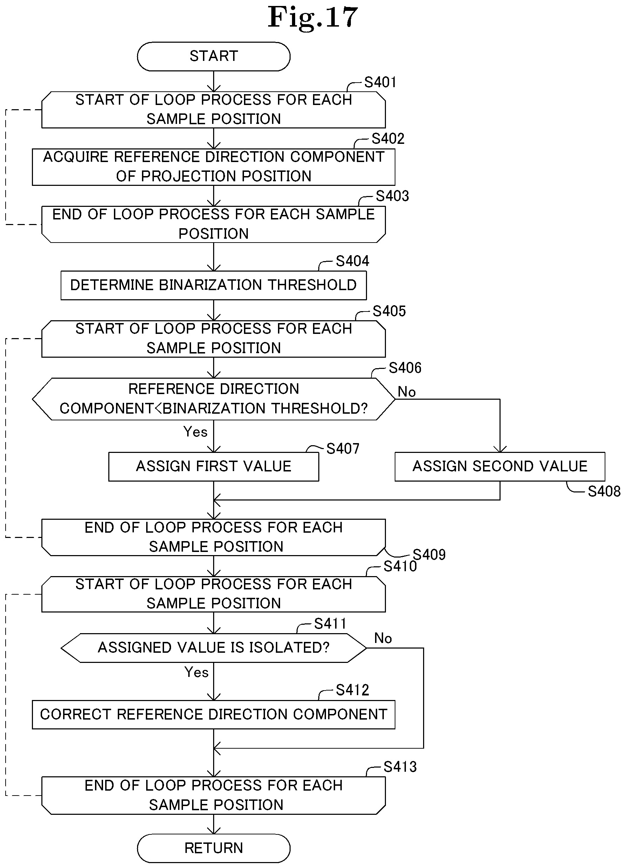

Next, the mounted body design assistance device 10 executes a projection position correction process (step S106 in Fig. 14). In this example, in the projection position correction process, the mounted body design assistance device 10 executes the third projection position correction process illustrated in Fig. 15, then executes the first projection position correction process illustrated in Fig. 16, and then executes the second projection position correction process illustrated in Fig. 17.

Alternatively, the mounted body design assistance device 10 may execute the second projection position correction process before the first projection position correction process.

As illustrated in Fig. 15, in the third projection position correction process, the mounted body design assistance device 10 executes a loop process (steps S201 to S205) in which each of the N sample positions SP-1 to SP-N is used as a processing target one by one in sequence.

In this loop process, the mounted body design assistance device 10 determines whether or not the biological component surface BS is present on the straight line (in other words, projection line), which passes through the focused sample position SP-n, which is the sample position SP-n being the processing target, and extends in the reference direction RD (step S202 in Fig. 15).

When the biological component surface BS is present on the projection line, the mounted body design assistance device 10 determines "Yes" in step S202, and stores presence information indicating that the biological component surface BS is present in association with the focused sample position SP-n (step S203 in Fig. 15).

In addition, when the biological component surface BS is not present on the projection line, the mounted body design assistance device 10 determines "No" in step S202, and stores absence information indicating that the biological component surface BS is not present in association with the focused sample position SP-n (step S204 in Fig. 15).

Next, the mounted body design assistance device 10 executes the above loop process (steps S201 to S205) for all of the N sample positions SP-1 to SP-N, and then proceeds to step S206.

Next, the mounted body design assistance device 10 executes a loop process (steps S206 to S208) in which each of absence sample positions SP-n, which are sample positions associated with the absence information in step S204 among the N sample positions SP-1 to SP-N, is used as a processing target one by one in sequence.

In this loop process, the mounted body design assistance device 10 determines the projection position PP-n for the focused sample position SP-n, which is the absence sample position SP-n being the processing target, by setting the average value of the reference direction components of projection positions (in other words, presence projection positions) for presence sample positions, which are sample positions associated with the presence information in step S204, among the extended adjacent projection positions for the focused sample position SP-n as the reference direction component of the projection position PP-n for the focused sample position SP-n (step S207 in Fig. 15).

Next, the mounted body design assistance device 10 executes the above loop process (steps S206 to S208) for all of the absence sample positions SP-n, and then ends the process of Fig. 15.

Next, as illustrated in Fig. 16, in the first projection position correction process, the mounted body design assistance device 10 executes a loop process (steps S301 to S303) in which each of the N sample positions SP-1 to SP-N is used as a processing target one by one in sequence.

In this loop process, the mounted body design assistance device 10 acquires the reference direction component of the projection position PP-n for the focused sample position SP-n, which is the sample position SP-n being the processing target (step S302 in Fig. 16).

Next, the mounted body design assistance device 10 executes the above loop process (steps S301 to S303) for all of the N sample positions SP-1 to SP-N, and then proceeds to step S304.

Next, the mounted body design assistance device 10 determines the reference value, the first threshold, and the second threshold based on the reference direction components of the N projection positions PP-1 to PP-N acquired for the N sample positions SP-1 to SP-N, respectively, in step S302 (step S304 in Fig. 16).

Next, the mounted body design assistance device 10 executes a loop process (steps S305 to S312) in which each of the N sample positions SP-1 to SP-N is used as a processing target one by one in sequence.

In this loop process, the mounted body design assistance device 10 acquires the reference direction component difference which is a value acquired by subtracting the reference value determined in step S304 from the reference direction component of the projection position PP-n for the focused sample position SP-n, which is the sample position SP-n being the processing target (step S306 in Fig. 16).

Next, the mounted body design assistance device 10 determines whether or not the reference direction component difference acquired in step S306 is smaller than 0 (step S307 in Fig. 16).

When the reference direction component difference is smaller than 0, the mounted body design assistance device 10 determines "Yes" in step S307, and determines whether or not the magnitude of the reference direction component difference is larger than the first threshold determined in step S304 (step S308 in Fig. 16).

When the magnitude of the reference direction component difference is larger than the first threshold, the mounted body design assistance device 10 determines "Yes" in step S308, and stores correction necessity information indicating that the projection position PP-n for the focused sample position SP-n needs to be corrected in association with the focused sample position SP-n (step S309 in Fig. 16).

In addition, when the magnitude of the reference direction component difference is equal to or smaller than the first threshold, the mounted body design assistance device 10 determines "No" in step S308, and stores correction unnecessity information indicating that the projection position PP-n for the focused sample position SP-n does not need to be corrected in association with the focused sample position SP-n (step S310 in Fig. 16).

Also, when the reference direction component difference is equal to or greater than 0, the mounted body design assistance device 10 determines "No" in step S307, and determines whether or not the magnitude of the reference direction component difference is greater than the second threshold determined in step S304 (step S311 in Fig. 16).

When the magnitude of the reference direction component difference is greater than the second threshold, the mounted body design assistance device 10 determines "Yes" in step S311, and stores correction necessity information indicating that the projection position PP-n for the focused sample position SP-n needs to be corrected in association with the focused sample position SP-n (step S309 in Fig. 16).

Also, when the magnitude of the reference direction component difference is equal to or less than the second threshold, the mounted body design assistance device 10 determines "No" in step S311, and stores correction unnecessity information indicating that the projection position PP-n for the focused sample position SP-n does not need to be corrected in association with the focused sample position SP-n (step S310 in Fig. 16).

Next, the mounted body design assistance device 10 executes the above loop process (steps S305 to S312) for all of the N sample positions SP-1 to SP-N, and then proceeds to step S313.

Next, the mounted body design assistance device 10 executes a loop process (steps S313 to S315) in which each of the correction necessity sample positions SP-n, which are sample positions SP-n associated with the correction necessity information in step S309 among the N sample positions SP-1 to SP-N, is used as a processing target one by one in sequence.

In this loop process, the mounted body design assistance device 10 corrects the projection position PP-n for the focused sample position SP-n, which is the correction necessity sample position SP-n being the processing target, by setting the average value of the reference direction components of projection positions (in other words, correction unnecessity projection positions) for sample positions associated with the correction unnecessity information among the extended adjacent projection positions for the focused sample position SP-n as the reference direction component of the projection position PP-n for the focused sample position SP-n (step S314 in Fig. 16).

Next, the mounted body design assistance device 10 executes the above loop process (steps S313 to S315) for all of the correction necessity sample positions SP-n, and then ends the process of Fig. 16.

Next, as illustrated in Fig. 17, in the second projection position correction process, the mounted body design assistance device 10 executes a loop process (steps S401 to S403) in which each of the N sample positions SP-1 to SP-N is used as a processing target one by one in sequence.

In this loop process, the mounted body design assistance device 10 acquires the reference direction component of the projection position PP-n for the focused sample position SP-n, which is the sample position SP-n being the processing target (step S402 in Fig. 17).

Next, the mounted body design assistance device 10 executes the above loop process (steps S401 to S403) for all of the N sample positions SP-1 to SP-N, and then proceeds to step S404.

Next, the mounted body design assistance device 10 determines the binarization threshold based on the reference direction components of the N projection positions PP-1 to PP-N acquired for the N sample positions SP-1 to SP-N, respectively, in step S402 (step S404 in Fig. 17).

Next, the mounted body design assistance device 10 executes a loop process (steps S405 to S409) in which each of the N sample positions SP-1 to SP-N is used as a processing target one by one in sequence.

In this loop process, the mounted body design assistance device 10 determines whether or not the reference direction component of the projection position PP-n for the focused sample position SP-n, which is the sample position SP-n being the processing target, is smaller than the binarization threshold determined in step S404 (step S406 in Fig. 17).

When the reference direction component of the projection position PP-n is smaller than the binarization threshold, the mounted body design assistance device 10 determines "Yes" in step S406, and assigns the first value to the focused sample position SP-n by storing first value information representing the first value in association with the focused sample position SP-n (step S407 in Fig. 17).

In addition, when the reference direction component of the projection position PP-n is equal to or greater than the binarization threshold, the mounted body design assistance device 10 determines "No" in step S406, and assigns the second value to the focused sample position SP-n by storing second value information representing the second value in association with the focused sample position SP-n (step S408 in Fig. 17).

Next, the mounted body design assistance device 10 executes the above loop process (steps S405 to S409) for all of the N sample positions SP-1 to SP-N, and then proceeds to step S410.

Next, the mounted body design assistance device 10 executes a loop process (steps S410 to S413) in which each of the N sample positions SP-1 to SP-N is used as a processing target one by one in sequence.

In this loop process, the mounted body design assistance device 10 determines whether or not the value assigned to the focused sample position SP-n, which is the sample position SP-n being the processing target, is isolated (step S411 in Fig. 17).

When the value assigned to the focused sample position SP-n is isolated, the mounted body design assistance device 10 determines "Yes" in step S411, and corrects the projection position PP-n for the focused sample position SP-n by setting the average value of the reference direction components of all extended adjacent projection positions for the focused sample position SP-n as the reference direction component of the projection position PP-n for the focused sample position SP-n (step S412 in Fig. 17).

Also, when the value assigned to the focused sample position SP-n is not isolated, the mounted body design assistance device 10 determines "No" in step S411, and proceeds to step S413 without executing the process of step S412.

Next, the mounted body design assistance device 10 executes the above loop process (steps S410 to S413) for all of the N sample positions SP-1 to SP-N, and then ends the process of Fig. 17.

In this manner, the mounted body design assistance device 10 executes the projection position correction process in step S106 in Fig. 14.

Next, the mounted body design assistance device 10 acquires the auxiliary position information based on the projection position information representing the N projection positions PP-1 to PP-N corrected in step S106 (step S107 in Fig. 14).

Next, the mounted body design assistance device 10 acquires the smooth surface information based on the projection position information representing the N projection positions PP-1 to PP-N corrected in step S106 and the auxiliary position information acquired in step S107 (step S108 in Fig. 14).

Next, the mounted body design assistance device 10 acquires the first moving solid body information and the second moving solid body information based on the smooth surface information acquired in step S108, and based on the position information and the reference direction information received in step S103 (step S109 in Fig. 14).

Next, the mounted body design assistance device 10 generates the intersection solid body information based on the first moving solid body information and the second moving solid body information acquired in step S109 (step S110 in Fig. 14).

Next, the mounted body design assistance device 10 generates the mounted body shape information based on the intersection solid body information generated in step S110 (step S111 in Fig. 14).

Next, the mounted body design assistance device 10 ends the process of Fig. 14.

When the process abnormality condition is satisfied in step S105, the mounted body design assistance device 10 determines "Yes" in step S105, and proceeds to step S112. Next, the mounted body design assistance device 10 outputs the abnormality notification information via the output device 14. Next, the mounted body design assistance device 10 ends the process of Fig. 14 without executing the process from step S106 to step S111.

As described above, the mounted body design assistance device 10 of the first embodiment assists in designing a mounted body to be mounted to a biological component surface BS, which is a surface of a part constituting a living body.

The mounted body design assistance device 10 includes a reference direction information reception unit 102, a position information reception unit 103, a projection position information acquisition unit 104, a smooth surface information acquisition unit 105, and a mounted body shape information generation unit 106.

The reference direction information reception unit 102 receives reference direction information representing a reference direction RD that is a direction of reference with respect to the biological component surface BS.

The position information reception unit 103 receives position information representing a position of a mounted body corresponding area AR, which is an area corresponding to the mounted body in a reference plane PL that is a plane perpendicular to the reference direction RD.

The projection position information acquisition unit 104 acquires projection position information representing projection positions PP-1 to PP-N, where the sample positions SP-1 to SP-N in the mounted body corresponding area AR on the reference plane PL are projected onto the biological component surface BS in the reference direction RD, respectively, based on biological component surface shape information representing a three-dimensional shape of the biological component surface BS, the received reference direction information, and the received position information.

The smooth surface information acquisition unit 105 acquires smooth surface information representing a smooth surface SS that is a smooth curved surface defined by using the projection positions PP-1 to PP-N as control points based on the acquired projection position information.

The mounted body shape information generation unit 106 generates mounted body shape information representing a three-dimensional shape of the mounted body so that the mounted body has at least a part of the smooth surface SS based on the acquired smooth surface information.

According to this, the user of the mounted body design assistance device 10 can easily generate the mounted body shape information by inputting the reference direction information and the position information to the mounted body design assistance device 10.Therefore, the mounted body can be easily designed according to the reference direction RD and the position of the mounted body corresponding area AR.

Moreover, according to the mounted body design assistance device 10, the mounted body shape information is generated so that the mounted body has at least a part of the smooth surface SS. Therefore, the design of the mounted body that can sufficiently fit the biological component surface BS can be assisted.

Furthermore, in the mounted body design assistance device 10 of the first embodiment, the mounted body shape information generation unit 106 generates intersection solid body information representing a three-dimensional shape of an intersection solid body CC generated by intersecting a first moved solid body MC1 formed by moving the smooth surface SS by a first distance along the reference direction RD, and a second moved solid body MC2 formed by moving the mounted body corresponding area AR by a second distance longer than the first distance along the reference direction RD, and generates the mounted body shape information based on the generated intersection solid body information.

According to this, the mounted body, which has the shape of the mounted body corresponding area AR when the mounted body is viewed in the reference direction RD, and is a plate-like body that fits the biological component surface BS, can be easily designed. Therefore, for example, a bone plate, which is mounted to a surface of a bone including a fractured portion in order to fix the fractured portion, can be easily designed.

Furthermore, in the mounted body design assistance device 10 of the first embodiment, the mounted body corresponding area AR is rectangular, and the sample positions SP-1 to SP-N are located in a grid pattern.

According to this, by adjusting the distance between sample positions SP-n adjacent to each other, the degree to which the smooth surface SS fits the biological component surface BS (in other words, the fitness degree of the smooth surface SS for the biological component surface BS) can be easily adjusted.

Furthermore, in the mounted body design assistance device 10 of the first embodiment, the smooth surface information acquisition unit 105 uses, for each of four sides constituting an outer edge of the mounted body corresponding area AR, auxiliary positions SU-1, SU-2, SU-3, SD-13, SD-14, SD-15, SR-3, SR-6, SR-9, SR-12, SR-15, SL-1, SL-4, SL-7, SL-10, SL-13, SC-1, SC-3, SC-13, SC-15 acquired by moving a projection position PP-n for a sample position SP-n located on the side in an extension direction, which is perpendicular to each of the side and the reference direction RD and is toward the outside of the mounted body corresponding area AR, as the control points in addition to the projection positions PP-1 to PP-N.

This allows the fitness degree of the smooth surface SS for the biological component surface BS in the vicinity of the outer edge of the mounted body corresponding area AR to be improved.

Furthermore, in the mounted body design assistance device 10 of the first embodiment, the projection position information acquisition unit 104 corrects a projection position PP-n for a focused sample position SP-n, which is one of the sample positions SP-1 to SP-N, based on a projection position for a sample position adjacent to the focused sample position SP-n when a magnitude of a difference between a component in the reference direction RD of the projection position PP-n for the focused sample position SP-n, and a reference value is greater than a threshold.

By the way, if there is a projection position PP-n where the magnitude of the difference between the component in the reference direction RD and the reference value is too large, there is a risk that the fitness degree of the smooth surface SS for the biological component surface BS becomes excessively low.

On the other hand, the mounted body design assistance device 10 can correct a projection position PP-n with an excessive difference between the component in the reference direction RD of the projection position PP-n and the reference value. Therefore, the fitness degree of the smooth surface SS for the biological component surface BS can be prevented from being excessively low.

Furthermore, in the mounted body design assistance device 10 of the first embodiment, the projection position information acquisition unit 104 assigns one of two values being a first value or a second value to each of the sample positions SP-1 to SP-N by binarizing components in the reference direction RD of the projection positions PP-1 to PP-N for the sample positions SP-1 to SP-N, and corrects a projection position PP-n for a focused sample position SP-n, which is one of the sample positions SP-1 to SP-N, based on a projection position for a sample position adjacent to the focused sample position SP-n when a value different from a value assigned to the focused sample position SP-n is assigned to all sample positions adjacent to the focused sample position SP-n.

By the way, if the projection position PP-n, whose component in the reference direction RD differs excessively from a projection position adjacent to the projection position PP-n, exists, there is a risk that the fitness degree of the smooth surface SS for the biological component surface BS becomes excessively low.

On the other hand, the mounted body design assistance device 10 can correct the projection position PP-n, whose component in the reference direction RD differs excessively from a projection position adjacent to the projection position PP-n. Therefore, the fitness degree of the smooth surface SS for the biological component surface BS can be prevented from being excessively low.

Furthermore, in the mounted body design assistance device 10 of the first embodiment, the projection position information acquisition unit 104 determines a projection position PP-n for a focused sample position SP-n, which is one of the sample positions SP-1 to SP-N, based on a projection position for a sample position adjacent to the focused sample position SP-n when the biological component surface BS is not present on a straight line that passes through the focused sample position SP-n and extends in the reference direction RD.

By the way, when the biological component surface BS is not present on the straight line, which passes through the sample position SP-n and extends in the reference direction RD, there is a risk that the fitness degree of the smooth surface SS for the biological component surface BS becomes excessively low if the projection position PP-n for the sample position SP-n is not appropriately set.

On the other hand, according to the mounted body design assistance device 10, when the biological component surface BS is not present on the straight line, which passes through the sample position SP-n and extends in the reference direction RD, the projection position PP-n for the sample position SP-n is determined based on a projection position adjacent to the projection position PP-n. Therefore, the fitness degree of the smooth surface SS for the biological component surface BS can be prevented from being excessively low.

Furthermore, the mounted body design assistance device 10 of the first embodiment includes an abnormality notification information output unit 107 that outputs abnormality notification information indicating that the mounted body shape information cannot be generated normally when a ratio of the number of sample positions SP-n, each of which a straight line extending in the reference direction RD passes through, the biological component surface BS not being present on the straight line, among the sample positions SP-1 to SP-N to a total number of the sample positions SP-1 to SP-N is greater than an abnormality threshold.

By the way, as the ratio of the number of sample positions SP-n, each of which a straight line extending in the reference direction RD passes through, the biological component surface BS not being present on the straight line, to the total number of sample positions SP-1 to SP-N becomes higher, the probability of generating the smooth surface SS that sufficiently fits the biological component surface BS becomes lower. Therefore, the mounted body design assistance device 10 can make the user aware of the possibility that the mounted body may not sufficiently fit the biological component surface BS.

This invention is not limited to the embodiments described above. For example, various modifications that can be understood by those skilled in the art may be made to the embodiment described above within the scope without departing from the spirit of the present invention.

### Reference Signs List

10 MOUNTED BODY DESIGN ASSISTANCE DEVICE
11 PROCESSING DEVICE
12 STORAGE DEVICE
13 INPUT DEVICE
14 OUTPUT DEVICE
101 BIOLOGICAL COMPONENT SURFACE SHAPE INFORMATION GENERATION UNIT
102 REFERENCE DIRECTION INFORMATION RECEPTION UNIT
103 POSITION INFORMATION RECEPTION UNIT
104 PROJECTION POSITION INFORMATION ACQUISITION UNIT
105 SMOOTH SURFACE INFORMATION ACQUISITION UNIT
106 MOUNTED BODY SHAPE INFORMATION GENERATION UNIT
107 ABNORMALITY NOTIFICATION INFORMATION OUTPUT UNIT
AR MOUNTED BODY CORRESPONDING AREA
BS BIOLOGICAL COMPONENT SURFACE
BU1 BUS
CC INTERSECTION SOLID BODY
MC1 FIRST MOVING SOLID BODY
MC2 SECOND MOVING SOLID BODY
PL REFERENCE PLANE
PP-1 TO PP-N PROJECTION POSITION
RD REFERENCE DIRECTION
SC-1, SC-3, SC-13, SC-15 CORNER EXTENDED AUXILIARY POSITION
SP-1 TO SP-N SAMPLE POSITION
SR-3, SR-6, SR-9, SR-12, SR-15, SL-1, SL-4, SL-7, SL-10, SL-13
LONG SIDE EXTENDED AUXILIARY POSITION
SS SMOOTH SURFACE
SU-1, SU-2, SU-3, SD-13, SD-14, SD-15 SHORT SIDE EXTENDED AUXILIARY POSITION

## Claims

1. A mounted body design assistance device that assists in designing a mounted body to be mounted to a biological component surface, which is a surface of a part constituting a living body, the mounted body design assistance device comprising:
a reference direction information reception unit that receives reference direction information representing a reference direction that is a direction of reference with respect to the biological component surface;
a position information reception unit that receives position information representing a position of a mounted body corresponding area, which is an area corresponding to the mounted body in a reference plane that is a plane perpendicular to the reference direction;
a projection position information acquisition unit that acquires projection position information representing projection positions, where sample positions in the mounted body corresponding area on the reference plane are projected onto the biological component surface in the reference direction, respectively, based on biological component surface shape information representing a three-dimensional shape of the biological component surface, the received reference direction information, and the received position information;
a smooth surface information acquisition unit that acquires smooth surface information representing a smooth surface that is a smooth curved surface defined by using the projection positions as control points based on the acquired projection position information; and
a mounted body shape information generation unit that generates mounted body shape information representing a three-dimensional shape of the mounted body so that the mounted body has at least a part of the smooth surface based on the acquired smooth surface information.

2. The mounted body design assistance device according to claim 1, wherein
the mounted body shape information generation unit generates intersection solid body information representing a three-dimensional shape of an intersection solid body generated by intersecting a first moved solid body formed by moving the smooth surface by a first distance along the reference direction, and a second moved solid body formed by moving the mounted body corresponding area by a second distance longer than the first distance along the reference direction, and generates the mounted body shape information based on the generated intersection solid body information.

3. The mounted body design assistance device according to claim 1 or claim 2, wherein
the mounted body corresponding area is rectangular, and
the sample positions are located in a grid pattern.

4. The mounted body design assistance device according to claim 3, wherein
the smooth surface information acquisition unit uses, for each of four sides constituting an outer edge of the mounted body corresponding area, auxiliary positions acquired by moving a projection position for a sample position located on the side in an extension direction, which is perpendicular to each of the side and the reference direction and is toward the outside of the mounted body corresponding area, as the control points in addition to the projection positions.

5. The mounted body design assistance device according to claim 1 or claim 2, wherein
the projection position information acquisition unit corrects a projection position for a focused sample position, which is one of the sample positions, based on a projection position for a sample position adjacent to the focused sample position when a magnitude of a difference between a component in the reference direction of the projection position for the focused sample position, and a reference value is greater than a threshold.

6. The mounted body design assistance device according to claim 1 or claim 2, wherein
the projection position information acquisition unit assigns one of two values being a first value or a second value to each of the sample positions by binarizing components in the reference direction of the projection positions for the sample positions, and corrects a projection position for a focused sample position, which is one of the sample positions, based on a projection position for a sample position adjacent to the focused sample position when a value different from a value assigned to the focused sample position is assigned to all sample positions adjacent to the focused sample position.

7. The mounted body design assistance device according to claim 1 or claim 2, wherein
the projection position information acquisition unit determines a projection position for a focused sample position, which is one of the sample positions, based on a projection position for a sample position adjacent to the focused sample position when the biological component surface is not present on a straight line that passes through the focused sample position and extends in the reference direction.

8. The mounted body design assistance device according to claim 1 or claim 2, comprising:
an abnormality notification information output unit that outputs abnormality notification information indicating that the mounted body shape information cannot be generated normally when a ratio of the number of sample positions, each of which a straight line extending in the reference direction passes through, the biological component surface not being present on the straight line, among the sample positions to a total number of the sample positions is greater than an abnormality threshold.

9. A mounted body design assistance method that assists in designing a mounted body to be mounted to a biological component surface, which is a surface of a part constituting a living body, the mounted body design assistance method including:
receiving reference direction information representing a reference direction that is a direction of reference with respect to the biological component surface;
receiving position information representing a position of a mounted body corresponding area, which is an area corresponding to the mounted body in a reference plane that is a plane perpendicular to the reference direction;
acquiring projection position information representing projection positions, where sample positions in the mounted body corresponding area on the reference plane are projected onto the biological component surface in the reference direction, respectively, based on biological component surface shape information representing a three-dimensional shape of the biological component surface, the received reference direction information, and the received position information;
acquiring smooth surface information representing a smooth surface that is a smooth curved surface defined by using the projection positions as control points based on the acquired projection position information; and
generating mounted body shape information representing a three-dimensional shape of the mounted body so that the mounted body has at least a part of the smooth surface based on the acquired smooth surface information.

10. A mounted body design assistance program that causes a computer to execute a process for assisting in designing a mounted body to be mounted to a biological component surface, which is a surface of a part constituting a living body, the process including:
receiving reference direction information representing a reference direction that is a direction being a reference with respect to the biological component surface;
receiving position information representing a position of a mounted body corresponding area, which is an area corresponding to the mounted body in a reference plane that is a plane perpendicular to the reference direction;
acquiring projection position information representing projection positions, where sample positions in the mounted body corresponding area on the reference plane are projected onto the biological component surface in the reference direction, respectively, based on biological component surface shape information representing a three-dimensional shape of the biological component surface, the received reference direction information, and the received position information;
acquiring smooth surface information representing a smooth surface that is a smooth curved surface defined by using the projection positions as control points based on the acquired projection position information; and
generating mounted body shape information representing a three-dimensional shape of the mounted body so that the mounted body has at least a part of the smooth surface based on the acquired smooth surface information.
